# FASCICULE DE BREVET EUROPEEN

(11) **EP 4 605 942 B1**
(45) Date de publication et mention de la délivrance du brevet: **07.10.2026**
(21) Numéro de dépôt: 23793767.7
(22) Date de dépôt: 19.10.2023
(51) Int. Cl.: G16C 20/20

(54) **PROCEDE DE CARACTERISATION D'UN PRODUIT PRESENTANT UNE ACTIVITE ANTIOXYDANTE, ET SYSTEME DE MISE EN OEUVRE DE CE PROCEDE**
VERFAHREN ZUR CHARAKTERISIERUNG EINES PRODUKTS MIT ANTIOXIDATIVER WIRKUNG UND SYSTEM ZUR DURCHFÜHRUNG DIESES VERFAHRENS
METHOD FOR CHARACTERISING A PRODUCT HAVING ANTIOXIDANT ACTIVITY, AND SYSTEM FOR IMPLEMENTING THIS METHOD

(30) Priorité: 20.10.2022 FR 2210844
(43) Date de publication de la demande: 27.08.2025
(73) Titulaire: Oxystress Technologies, 54230 Neuves-Maisons (FR)
(72) Inventeur: MEZIANE, Smail, 54230 Neuves-Maisons (FR)
(74) Mandataire: IP Trust
(86) Numéro de dépôt international: PCT/EP2023/079116
(87) Numéro de publication internationale: WO 2024/083968

(56) Documents cités:
- SHARPE ERICA ET AL: "Metal oxide based multisensor array and portable database for field analysis of antioxidants", SENSORS AND ACTUATORS B: CHEMICAL, ELSEVIER BV, NL, vol. 193, 11 December 2013 (2013-12-11), pages 552 - 562, XP028818813, ISSN: 0925-4005, DOI: 10.1016/J.SNB.2013.11.088
- GOMEZ-ROMERO M ET AL: "Automated identification of phenolics in plant-derived foods by using library search approach", FOOD CHEMISTRY, ELSEVIER LTD, NL, vol. 124, no. 1, 1 January 2011 (2011-01-01), pages 379 - 386, XP027183510, ISSN: 0308-8146, [retrieved on 20100616]
- FERNANDEZ-PACHON M S ET AL: "Determination of the phenolic composition of sherry and table white wines by liquid chromatography and their relation with antioxidant activity", ANALYTICA CHIMICA ACTA, ELSEVIER, AMSTERDAM, NL, vol. 563, no. 1-2, 23 March 2006 (2006-03-23), pages 101 - 108, XP027912174, ISSN: 0003-2670, [retrieved on 20060323]
- PROTEGGENTE A R ET AL: "The antioxidant activity of regularly consumed fruit and vegetables reflects their phenolic and vitamin C composition", FREE RADICAL RESEARCH, TAYLOR & FRANCIS, GB, vol. 36, no. 2, 1 January 2002 (2002-01-01), pages 217 - 233, XP002982135, ISSN: 1071-5762, DOI: 10.1080/10715760290006484
- HEO ET AL: "Antioxidant capacities of individual and combined phenolics in a model system", FOOD CHEMISTRY, ELSEVIER LTD, NL, vol. 104, no. 1, 14 April 2007 (2007-04-14), pages 87 - 92, XP022040319, ISSN: 0308-8146, DOI: 10.1016/J.FOODCHEM.2006.11.002
- KARAMAN ^ ET AL: "Comparison of total antioxidant capacity and phenolic composition of some apple juices with combined HPLCCUPRAC assay", FOOD CHEMISTRY, ELSEVIER LTD, NL, vol. 120, no. 4, 15 June 2010 (2010-06-15), pages 1201 - 1209, XP026877619, ISSN: 0308-8146, [retrieved on 20091202]
- MUNTEANU IRINA GEORGIANA ET AL: "Analytical Methods Used in Determining Antioxidant Activity: A Review", INTERNATIONAL JOURNAL OF MOLECULAR SCIENCES, vol. 22, no. 7, 25 March 2021 (2021-03-25), Basel, CH, XP093040894, ISSN: 1661-6596, DOI: 10.3390/ijms22073380
- GULCIN ILHAMI: "Antioxidants and antioxidant methods: an updated overview", ARCHIVES OF TOXICOLOGY, SPRINGER, DE, vol. 94, no. 3, 1 March 2020 (2020-03-01), pages 651 - 715, XP037071782, ISSN: 0340-5761, [retrieved on 20200316], DOI: 10.1007/S00204-020-02689-3

## Description

### Domaine technique

Le domaine de l'invention est celui des produits alimentaires, compléments alimentaires ou pharmaceutiques présentant une activité antioxydante, et plus spécifiquement celui de la détermination de la composition d'un tel produit en molécules lui apportant son activité antioxydante.

### Technique antérieure

Une molécule antioxydante est une molécule qui ralentit ou empêche l'oxydation d'autres substances chimiques à son contact.

Dans le domaine de la santé et de l'industrie alimentaire, les molécules antioxydantes et leurs effets représentent un champ de recherche actif, ces molécules étant capable de limiter le stress oxydatif au niveau cellulaire pour un individu alors que ce stress oxydatif peut devenir pathologique lorsqu'il est excessif, par exemple lorsque des espèces telles que ROS ou RNS (respectivement Reactive Oxygen Species et Reactive Nitrogen Species en terminologie anglaise) sont produite en excès de l'organisme.

Dans ce contexte, on considère que la prise d'antioxydants sous forme pharmaceutique, cosmétique, alimentaire, ou de compléments alimentaires comme susceptible de limiter les dommages liés aux ROS et NRS en excès et ainsi prévenir la survenue de diverses maladies telles que troubles neurodégénératifs (maladie de Parkinson, maladie d'Alzheimer, ischémie cérébrale), maladies cardiovasculaires (athérosclérose et hypertension), diabète sucré, maladies respiratoires (asthme), développement de la cataracte et cancers (cancers colorectal, de la prostate, du sein, du poumon, de la vessie).

On peut se référer à la publication de Qader et al., « Natural Nrf2 Activators from Juices, Wines, Coffee, and Cocoa », Beverages 2020, 6(4), 68.

Parmi les molécules antioxydantes, on peut citer en particulier les polyphénols, qui comprennent des flavonoïdes et des non-flavonoïdes ainsi que d'autres composés phénoliques.

Les polyphénols sont responsables du pouvoir antioxydant de nombreuses boissons, avec, pour donner quels exemples de cette grande famille de composés, l'acide chlorogenic pour le café, quercétine, l'acide chlorogénique et le resvératrol pour le vin, les catéchines et les acides phénoliques pour la bière, la quercétine et l'acide chlorogénique pour le thé ou encore la quercétine, l'acide chlorogénique et le 4-éthyl catéchol pour les jus de fruits.

Ainsi, les producteurs ou les consommateurs de ces produits souhaitent connaître plus précisément leurs caractéristiques concernant leurs activités antioxydantes à des fins prophylactiques ou curatives.

Cependant, un seul produit, un vin rouge par exemple, peut comprendre plusieurs dizaines de molécules phénoliques antioxydantes (anthocyanines, dihydroflavonols, flavanoles, flavanones, flavonoïdes, tyrosol etc...) participant chacune à l'activité antioxydante globale du vin considéré.

En outre, la variété et le mode de culture du raisin, le mode de vinification ou encore la durée et les conditions de stockages (température, exposition à la lumière, humidité...) influent sur la composition du vin.

Les méthodes conventionnelles d'analyse du vin consistent à doser une ou plusieurs molécules spécifiques, ce qui requiert un niveau de compétence et de spécialisation que peu de laboratoires possèdent, des délais d'analyse pouvant en pratique se compter en semaines, ainsi que des coûts très élevés, rapidement prohibitifs pour un producteur qui souhaiterait réaliser un suivi de la présence de plusieurs molécules dans son produit.

Alternativement, certaines technologies telles que la technologie PAOT^{®} sont capable de mesurer rapidement et à coût réduit l'activité antioxydante totale d'un produit par des méthodes électrochimiques, comme détaillé dans la demande de brevet FR 3 089 011.

Il s'agit cependant d'une mesure globale sur un produit donné et ce type de mesure ne permet pas par elle-même de détailler la composition d'un produit quelconque en ses composés antioxydants.

Nous voyons donc qu'il existe un besoin non satisfait d'une méthode de détermination des composés antioxydants d'un produit quelconque qui soit rapide, bon marché et accessible à un grand nombre d'utilisateurs sans se limiter à quelques professionnels qualifiés.

La publication d'Erica Sharpe et al., "Metal oxide based multisensor array and portable database for field analysis of antioxidants", Sensors and Actuators B: Chemical, Volume 193, 2014 présente un procédé de détection de polyphénol et de caractérisation d'échantillons contenant un antioxydant.

### Exposé de l'invention

Un but de l'invention est de fournir un procédé capable de mener à la détermination des concentrations de composés présentant une activité antioxydante, ou composés antioxydants, dans un produit quelconque présentant une activité antioxydante.

A ces effets, l'invention concerne un procédé de caractérisation d'un produit-cible à caractériser présentant une activité antioxydante, comprenant une étape de réception d'un signal représentatif d'une mesure d'une activité antioxydante globale du produit-cible à caractériser, le procédé étant caractérisé en ce qu'il comprend en outre les étapes de recevoir, au moyen d'une unité de traitement informatique, un signal représentatif d'un type du produit-cible à caractériser ; identifier dans une base de données un produit de référence correspondant au produit-cible à caractériser sur la base du signal représentatif d'un type du produit à caractériser ; récupérer dans la base de donnée des identifiants de constituants antioxydants du produit-cible à caractériser sur la base du signal représentatif d'un type du produit à caractériser ; récupérer dans la base de donnée, au moyen de l'unité de traitement informatique, (a) des coefficients respectivement représentatifs des activités antioxydantes des constituants antioxydants dans le produit-cible à caractériser, et (b) des activités antioxydantes individuelles des constituants considérés individuellement ; et calculer, au moyen de l'unité de traitement informatique, des concentrations des constituants antioxydants dans le produit-cible à caractériser à partir du signal représentatif d'une activité antioxydante globale du produit-cible, des coefficients, et des activités antioxydantes à partir de l'équation $C {′}_{j} = \frac{{Coef}_{j} \times {AA}_{GlobMes}}{{AA}_{j}}$ dans laquelle *C^{'}ⱼ* représente les concentrations des constituants antioxydants dans le produit-cible, *Coefⱼ* indique les contributions relatives des constituants à l'activité antioxydante globale du produit de référence, *AA_{GlobMes}* représente l'activité antioxydante globale du produit-cible et *AAⱼ* représente les activités antioxydantes individuelles des constituants considérés individuellement, et les contributions relatives des constituants à l'activité antioxydante indiquées par *Coefⱼ* sont tirées de l'équation ${Coef}_{j} = \frac{{C}_{j}}{{\sum }_{j} {C}_{j}}$ dans laquelle *Cⱼ* représente les concentrations respectives des constituants dans le produit de référence (Prodᵢ).

Ce procédé peut être mis en œuvre au moyen de la technologie PAOT^{®} ou de toute autre technologie permettant de mesurer l'activité antioxydante globale d'un produit.

Un premier avantage des ce procédé est que, au niveau de l'utilisateur final, une seule mesure de l'activité antioxydante globale d'un produit permet de déduire l'ensemble des concentrations des constituants de ce produit qui participent à l'activité antioxydante de ce dernier.

Un second avantage est qu'aucune expertise particulière n'est nécessaire à la mise en œuvre du procédé, les aspects du procédé nécessitant de l'expertise étant reportés dans les phases préparatoires à la détermination des concentrations de constituants antioxydants du produit à caractériser.

Des avantages supplémentaires, découlant directement du procédé, consistent en un coût réduit, une grande flexibilité et une grande réactivité du procédé, puisque ne nécessitant de la part de l'utilisateur que la préparation d'un échantillon unique pour une mesure unique pouvant être réalisé très simplement au moyen d'un dispositif de mesure électrochimique de l'activité antioxydante totale d'un produit telle que la technologie PAOT^{®}.

Ainsi, la mise en œuvre du procédé selon l'invention, flexible et de coût modeste, permet une suivi de la production de produits tels que le vin, les fruits et leurs jus, le thé, le café de manière par exemple à optimiser leur composition en vue de prévenir ou soigner des maladies liées au stress oxydatif.

Selon d'autres caractéristiques non limitatives de l'invention, prises seules, ou selon toute combinaison techniquement réalisable :
- le calcul peut n'être effectué de manière à ne considérer que des constituants antioxydants dont les activités antioxydantes individuelles multipliées par leurs concentrations respectives dépassent 5%, de l'activité antioxydante globale du produit de référence ou du produit-cible à caractériser ;
- le calcul peut n'être effectué de manière à ne considérer que des constituants antioxydants dont la somme des activités antioxydantes pondérées par leurs concentrations respectives dépassent 80% de l'activité antioxydante globale du produit de référence ou du produit-cible à caractériser ; et
- le procédé peut comprendre les étapes de mesurer l'activité antioxydante globale du produit-cible au moyen d'un dispositif de mesure, et de générer le signal représentatif d'un type du produit-cible à caractériser sur la base d'une information stockée dans une mémoire informatique et le signal représentatif d'une activité antioxydante globale du produit-cible sur la base d'un résultat de la mesure.

L'invention s'étend à un système de caractérisation d'un produit-cible à caractériser présentant une activité antioxydante, le système comprenant une base de données et une unité de calcul configurés pour mettre en œuvre le procédé.

### BREVE DESCRIPTION DES FIGURES

D'autres caractéristiques et avantages de l'invention ressortiront de la description détaillée de l'invention qui va suivre en référence aux figures annexées, sur lesquelles :
[Fig.1] La [Fig.1] illustre un procédé selon l'invention ;
[Fig.2] La [Fig.2] illustre un système de mise en œuvre d'un procédé selon l'invention ; et
[Fig.3] La [Fig.3] représente un tableau illustrant la mise en œuvre du procédé de la [Fig.1].

### DESCRIPTION DETAILLEE DE L'INVENTION

Un procédé selon l'invention consiste, dans une phase de préparation, à procéder d'une part à des analyses chimiques de produits de manière à déterminer leurs compositions respectives en constituants ayant chacun une action antioxydante, et d'autre part à déterminer par mesure l'activité antioxydante de chacun de ces constituants considérés individuellement.

Dans une phase d'application, on procède à une mesure de l'analyse de l'activité antioxydante globale d'un produit-cible à caractériser, puis on remonte à la constitution de ce produit-cible en constituants antioxydants grâce aux données obtenues durant la phase de préparation et au résultat de la mesure.

Ces deux phases et un exemple de leurs mise en œuvre et des moyens associés sont détaillées ci-dessous.

### Phase de préparation

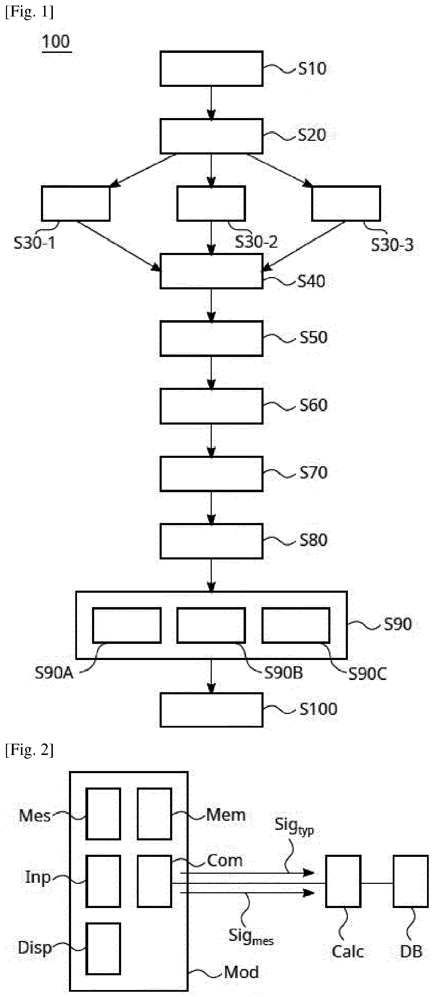

La phase de préparation comprend un travail de fond de collecte de données qui consiste tout d'abord à identifier et lister, dans une base de donnée informatique DB, des produits de référence ayant des propriétés antioxydantes en raison de leurs contenus en constituants antioxydants, au cours d'une étape S10 d'un procédé 100 de caractérisation d'un produit présentant une activité antioxydante illustré par la [Fig.1].

On connaît certains produits ayant des effets antioxydant dans le cadre d'une consommation alimentaire, tels que les vins, les bières, les thés les cafés ou encore certains jus de fruits, mais on peut également s'intéresser par exemple les caractéristiques antioxydantes de fluides biologiques (sang, urine...) ou de produits cosmétiques (crèmes, lotions...).

Des points de vue des producteurs, des fournisseurs, ou des consommateurs finaux, il serait intéressant de caractériser en particulier les contenus respectifs de ces produits en constituants antioxydants.

Dans le cas d'un producteur de vin, une telle caractérisation permettrait un suivi plus précis de l'influence des paramètre de production du vin, allant du type de raisin et de son mode de cultivation au processus de vinification et des conditions de stockage sur son contenu en un ou plusieurs constituants spécifiques.

A une étape S20, on identifie et associe à chacun des produits de références listés au cours de l'étape S10 les constituants respectifs qui leurs confèrent une activité antioxydante, par exemple sous forme d'un tableau stocké dans la base de données informatique DB.

Ces constituants sont désignés par des identifiants Constᵢⱼ indiquant un j^{ème} constituant d'un i^{ème} produit de référence Prodᵢ.

Cette étape peut être effectuée par une étude bibliographique des constituants antioxydants déjà identifiées.

Alternativement, on peut tester l'activité antioxydante des constituants des produits de référence identifiés au moyen de méthodes connues.

A une étape S30-1, on mesure les concentrations des constituants de chacun des produits de référence listés, à une étape S30-2, on mesure l'activité antioxydante des constituants pris isolément, et à une étape S30-3 optionnelle, on mesure l'activité antioxydante globale des produits de références listés.

Les mesures de concentrations et d'activités antioxydantes peuvent être réalisées par des méthodes conventionnelles.

Les mesures d'activités antioxydantes peuvent être réalisées en particulier au moyen de la technologie PAOT^{®}, qui consiste, dans le cas d'un travail sur un échantillon liquide, à mesurer les changements électrochimiques d'une solution aqueuse contenant une molécule M à l'état de radical libre au moyen d'une électrode de travail et d'une électrode de référence, suite à l'ajout d'un échantillon à analyser.

Ces changements s'expliquent par les variations des concentrations des formes oxydées / réduites de M en présence d'antioxydants, l'activité antioxydante étant obtenue en mesurant une diminution du potentiel électrochimique de la solution et est évaluée selon la formule suivante :
$activité antioxydante = \frac{EPproduct 10 - EPcontrol 0}{EPcontrol 0} \times 100 %$
où EPproduct10 est le potentiel électrochimique à la fin de l'analyse, c'est-à-dire lorsque le potentiel de la solution se stabilise (arrêt automatique de l'analyse) et EP-contrôle0 est le potentiel électrochimique au temps 0, instant de l'ajout de l'échantillon.

L'expression « activité antioxydante » désigne aussi bien un phénomène physico-chimique qu'une estimation numérique de cette activité que l'on pourrait reformuler comme « niveau d'activité antioxydante ».

Selon les produits considérés, la molécule M utilisée peut être de natures différentes, comme déterminée par le praticien.

On peut se référer à la publication de Joël Pincemail et al., « PAOT-Liquid® Technology: An Easy Electrochemical Method for Evaluating Antioxidant Capacity of Wines », Diseases 2019 Jan 21;7(1):10.

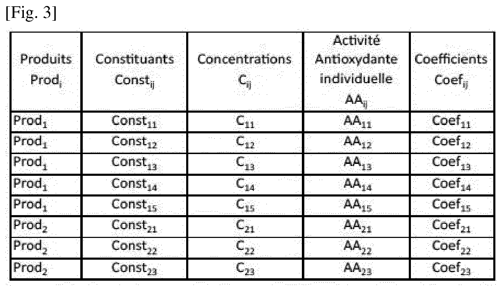

Les résultats des mesures des étapes S30-1 et S30-2 sont stockées dans la base de données DB, en association avec les constituants concernés, données pouvant par exemple être visualisées sous la forme du tableau représenté par la [Fig.3] comprenant les données correspondant à deux produits i Prod i, leurs constituants respectifs Constᵢⱼ, les concentrations Cᵢⱼ et les activités antioxydantes individuelles respectives AAᵢⱼ de chaque constituants j dans un produit i, i variant de 1 à 2 pour les deux produits considérés, j variant de 1 à 5 pour le produit 1, indiquant que 5 constituants sont associés ce produit 1, j variant de 1 à 3 pour le produit 2, indiquant que 3 constituants sont associés à ce produit 1, dans cet exemple.

A ce stade, on peut optionnellement introduire au moins un critère de sélection des constituants pour chacun des produits de manière à ne conserver dans la base de données BD que les constituants ayant une activité antioxydante significative pour le produit concerné, au cours d'une étape S40.

Alternativement, tous les constituants peuvent être conservés dans la bases de données, mais on ne tiendra compte dans les calculs subséquents que des constituants répondant au dit critère de sélection.

Des avantages de cette pratique sont que la base de données ne requiert par d'être exhaustive en ce qui concerne l'identification des constituants antioxydants de chacun des produits de référence pour être exploitable, ce qui peut considérablement réduire la somme de travail nécessaire pour l'alimenter en données et la rendre accessible pour la caractérisation de produits au sujet desquelles les connaissances ne sont pas encore complètes.

En outre une simplification des données prises en compte peut permettre une mise en œuvre plus simple du procédé et une meilleurs prise en main par un utilisateur non spécialiste.

Un premier critère de sélection possible consiste à ne considérer pour chaque produit de référence que les constituants antioxydants dont les activités antioxydantes pondérées par leurs concentrations respectives dépassent un seuil défini par l'utilisateur, par exemple 5%, préférablement 10%, de l'activité antioxydante globale du produit considéré.

Un deuxième critère de sélection possible consiste à ne considérer pour chaque produit de référence que les constituants antioxydants dont la somme des activités antioxydantes pondérées par leurs concentrations respectives, c'est-à-dire le produit de l'activité antioxydante par la concentration pour chacun des constituants considérés, dépassent 80%, préférablement 90%, de l'activité antioxydante globale du produit considéré.

Ces deux critères nécessitent la mesure de l'activité antioxydante globale du produit de référence considéré, qui peut être enregistrée dans la base de données DB.

Ces critère peuvent être cumulés, c'est-à-dire qu'un constituant donné d'un produit donné peut avoir à respecter un deux, ou trois des critères ci-dessus pour être retenu dans la base de donnée ou pour être pris en compte dans les calculs subséquents.

A une étape S50, en partant des données obtenues, on peut définir des coefficients Coefⱼ indiquant la contribution relative d'un constituant Constⱼ à l'activité antioxydante globale d'un produit de référence Prodᵢ selon l'équation 2, ${Coef}_{j} = \frac{{C}_{j}}{{\sum }_{j} {C}_{j}}$ Equation 2
puis les stocker dans la base de données DB, comme visible dans le tableau Tab 1.

Les étapes ci-dessus peuvent être réalisées progressivement, en laboratoire et de manières décentralisée de manière à enrichir peu à peu la base de données en produits listés en vue d'applications de plus en plus diverses et de résultats de plus en plus précis.

Les étapes suivantes sont à l'initiative d'un utilisateur final, qui mettra avantageusement à profit le résultat du travail de collecte de données et de calculs effectué au cours des étapes S10 à S50.

### Phase applicative

La phase applicative consiste, pour un utilisateur final, à mesurer l'activité antioxydante globale d'un produit-cible à caractériser ayant une activité antioxydante et d'un type listé dans la base de données DB, en vue de la détermination, à partir de cette seule mesure et des informations stockées dans la base de données, des concentrations des constituants antioxydants de ce produit-cible.

Un telle détermination est rendu possible par le fait que l'activité antioxydante globale d'un produit quelconque représente la somme des activité antioxydantes de chacun des ses composants pondérées par la concentration du composant constituant le produit, comme représenté par la formule générale Equation 1 :
${AA}_{Glob} \left(produit\right) = {\sum }_{k} {C}_{k} {AA}_{k}$ Equation 1 où *Cₖ* et *AAₖ* représentent respectivement la concentration et l'activité antioxydante d'un k^{ième} constituant du produit.

En conséquence, à partir (a) des données enregistrées dans la base de données DB qui indiquent la concentration et l'activité antioxydante des constituants d'un échantillon de référence d'un produit considéré, chaque constituant étant considéré individuellement, (b) de la relation donnée ci-dessus entre activité antioxydante globale et activité antioxydante individuelle de chacun des constituants du produit considéré et (c) de l'activité antioxydante globale d'un échantillon de mesure d'un produit-cible à caractériser de même nature que le produit considéré, on peut remonter à la compositions en constituants antioxydants de l'échantillon de mesure dudit produit-cible à caractériser.

Ainsi, une première étape S60 à réaliser par l'utilisateur final consiste à mesurer l'activité antioxydante globale d'un produit-cible à caractériser, qui donne un résultat AA_{GlobMes} ; cette mesure est enregistrée localement dans une mémoire informatique Mem par l'utilisateur, éventuellement automatiquement au moyen du dispositif de mesure employé.

La méthode de mesure employée pour déterminer l'activité antioxydante globale d'un produit-cible est de préférence la même que celle employée pour déterminer les activités antioxydantes des constituants des produits de références au cours de la phase préparatoire.

A une étape S70 suivant, ou précédant, l'étape S60, l'utilisateur entre dans la mémoire informatique Mem le type Typ du produit-cible, ou la nature de ce produit, dont il a mesuré l'activité antioxydante globale.

Il peut s'agit d'un type de vin, de café, de jus de fruit, de fluide biologique et, plus généralement, de tout échantillon dont une activité antioxydante est susceptible d'être mesurée.

L'utilisateur peut avoir à sa disposition un dispositif d'affichage Disp affichant une liste des produits de référence Prodᵢ mentionnés dans la base de données DB, et peut choisir dans cette liste un produit de référence correspondant au produit-cible par des moyens de saisie Inp selon des méthodes informatiques conventionnelles, de manière à associer au résultat de la mesure effectuée le type de produit sur lequel la mesure a été faite au sein de la mémoire informatique Mem.

Il est important pour l'utilisateur de choisir un produit de référence aussi proche que possible du produit-cible en termes de composition chimique : dans un produit de référence donné, la nature et les concentrations relatives des constituants antioxydants sont largement constants, et c'est ce qui permet de remonter de manière fiable à ces concentrations pour le produit-cible à caractériser à partir d'une seule mesure d'activité antioxydante globale.

De cette manière, on peut considérer que les coefficients Coefᵢⱼ déterminés à l'étape S50 pour un produit de référence sont respectivement représentatifs des activités antioxydantes des constituants Constᵢⱼ dans le produit-cible à caractériser.

L'utilisateur peut, par exemple, être un vigneron souhaitant caractériser un vin d'un cépage donné d'une certaine année par comparaison avec des données de référence obtenues pour le même vin d'une année précédente et enregistrées dans la base de données DB.

A une étape S80, un signal Sig_{typ} représentatif du type Typ du produit-cible à caractériser stocké dans la mémoire Mem et un signal Sigₘₑₛ représentatif du résultat de la mesure AA_{GlobMes} sont générés et transmis par une unité de communication Com à une unité de calcul et de traitement informatique Calc fonctionnellement reliée à la base de donnée DB.

Le dispositif Mes de mesure employé par l'utilisateur, la mémoire informatique Mem, le dispositif d'affichage Disp, les moyens de saisie Inp et l'unité de communication Com peuvent être intégrés au sein d'un Module de mesure Mod, et l'unité de calcul et de traitement informatique Calc et la base de données DB peuvent être distants du Module de mesure Mod, comme illustré par la [Fig.2].

En particulier, le base de donnée et l'unité de calcul et de traitement informatique peuvent être connectés au module de mesure Mod par l'intermédiaire d'un réseau informatique tel que le réseau internet.

Il ne s'agit cependant que d'une configuration prise en exemple, et on peut imaginer que la base de données et l'unité de calcul et de traitement informatique soient également intégrés au sein du module de mesure Mod, ou encore que la mesure soit réalisée de manière totalement indépendant de tout système intégré et que son résultat soit entré manuellement dans la mémoire informatique Mem.

On peut considéré un système Sys de mise en œuvre du procédé 100 sous une forme totalement intégré dans un appareillage unique ou, alternativement, comme un ensemble distribué d'appareillages physiquement distincts les uns des autres mais fonctionnellement connectés, par exemple au moyen d'un réseau informatique.

En tous les cas, l'invention ne se limite pas à une implémentation particulière des éléments matériels employés pour sa mise en œuvre,

A une étape S90, grâce à l'information sur le type de produit de référence transmise par le signal Sig_{typ}, l'unité de calcul et de traitement informatique Calc identifie (étape S90A) un produit de référence (Prodᵢ) de la base de données (DB) correspondant au produit à caractériser et récupère (étape S90B) dans la base de données DB les identifiants des constituants j Constᵢⱼ de ce produit de référence i ainsi que et les données associées, plus spécifiquement leurs coefficients respectifs Coefᵢⱼ et leurs activités antioxydantes individuelles AAᵢⱼ, puis calcule (étape S90C) les concentration *C^{'}ⱼ* de chacun des constituants j du produit-cible ayant une activité antioxydante au moyen de la formule équation 3 :
$C {′}_{j} = \frac{{Coef}_{j} \times {AA}_{GlobMes}}{{AA}_{j}}$ Eq. 3

Il est à noter que le calcul peut être effectué en ne considérant que les constituants répondants à un ou plusieurs des critères mentionnés à l'étape S40.

En outre, en ce qui concerne les deuxième et troisième critères de sélection, les seuils peuvent être calculés en pourcentages de l'activité antioxydante totale du produit de référence ou, alternativement en pourcentages de l'activité antioxydante totale du produit-cible qui vient d'être mesurée par l'utilisateur final.

A une étape S100, l'unité de calcul et de traitement informatique Calc revoie à l'unité de communication Com les identifiants Constᵢⱼ des constituants antioxydant et leurs concentrations respectives *C^{'}ⱼ* déduite de la mesure pour les stocker dans la mémoire Mem et les afficher sur le dispositif d'affichage Disp à destination de l'utilisateur.

Nous voyons que, à la différence d'une approche classique, qui nécessite un investissement lourd en termes de temps passé, de coût et de contact à prendre auprès de spécialistes, le procédé selon l'invention est rapide, simple, et accessible à un non-spécialiste.

La formule générale de l'équation 1 n'est rigoureusement exacte que si chacun des constituants antioxydants dans le produit A et leur activités antioxydantes respectives sont connus.

Toutefois, cette formule générale restera utilisable en dépit de la présence éventuelle de constituants antioxydants qui ne seraient pas connus ou bien d'un choix volontaire de ne conserver dans les calculs que les constituants ayant une activité antioxydante significative, soit parce que les constituants connus permettent une bonne approximation (on peut se référer aux critères de l'étape S40), soit parce ce sont les variations des caractéristiques d'une produit qui sont importantes et peuvent être effectivement étudiées même si des constituants antioxydants sont négligés, indépendamment de leurs parts relatives dans l'activité antioxydante globale du produit.

Bien entendu l'invention n'est pas limitée à la description ci-dessus, et on peut y apporter des variantes de réalisation sans sortir du cadre de l'invention tel que défini par les revendications.

## Revendications

1. Procédé (100) de caractérisation d'un produit-cible à caractériser présentant une activité antioxydante, comprenant une étape (S60) de réception d'un signal (Sigₘₑₛ) représentatif d'une mesure d'une activité antioxydante globale du produit-cible à caractériser, le procédé étant **caractérisé en ce qu'**il comprend en outre les étapes de :
- recevoir (S80), au moyen d'une unité de traitement informatique (Calc), un signal (Sig_{typ}) représentatif d'un type du produit-cible à caractériser ;
- identifier (S90A) dans une base de données (DB) un produit de référence (Prodᵢ) correspondant au produit-cible à caractériser sur la base du signal (Sig_{typ}) représentatif d'un type du produit à caractériser,
- récupérer (S90B) dans la base de donnée (DB) des identifiants (Constᵢⱼ) de constituants antioxydants du produit-cible à caractériser sur la base du signal (Sig_{typ}) représentatif d'un type du produit à caractériser ;
- récupérer (S90B) dans la base de donnée (DB), au moyen de l'unité de traitement informatique (Calc), (a) des coefficients (Coefᵢⱼ) respectivement représentatifs des activités antioxydantes des constituants antioxydants dans le produit-cible à caractériser, et (b) des activités antioxydantes individuelles (AAᵢⱼ) des constituants considérés individuellement ; et
- calculer (S90C), au moyen de l'unité de traitement informatique (Calc), des concentrations (*C^{'}ⱼ*) des constituants antioxydants (Constᵢⱼ) dans le produit-cible à caractériser à partir du signal (Sigₘₑₛ) représentatif d'une activité antioxydante globale du produit-cible, des coefficients (Coefᵢⱼ), et des activités antioxydantes (AAᵢⱼ) à partir de l'équation $C {′}_{j} = \frac{{Coef}_{j} \times {AA}_{GlobMes}}{{AA}_{j}}$ dans laquelle *C^{'}ⱼ* représente les concentrations des constituants antioxydants dans le produit-cible, *Coefⱼ* indique les contributions relatives des constituants à l'activité antioxydante globale du produit de référence (Prodᵢ), AA_{GlobMes} représente l'activité antioxydante globale du produit-cible et *AAⱼ* représente les activités antioxydantes individuelles des constituants considérés individuellement, et les contributions relatives des constituants à l'activité antioxydante indiquées par *Coefⱼ* sont tirées de l'équation ${Coef}_{j} = \frac{{C}_{j}}{{\sum }_{j} {C}_{j}}$ dans laquelle *Cⱼ* représente les concentrations respectives des constituants dans le produit de référence (Prodᵢ).

2. Le procédé selon la revendication 1, dans lequel le calcul (S90) est effectué de manière à ne considérer que des constituants antioxydants dont les activités antioxydantes individuelles (AAᵢⱼ) multipliées par leurs concentrations respectives (Cᵢⱼ) dépassent 5%, de l'activité antioxydante globale du produit de référence ou du produit-cible à caractériser.

3. Le procédé selon la revendication 1, dans lequel le calcul (S90) est effectué de manière à ne considérer que des constituants antioxydants dont la somme des activités antioxydantes pondérées par leurs concentrations respectives dépassent 80% de l'activité antioxydante globale du produit de référence ou du produit-cible à caractériser.

4. Le procédé selon l'une quelconque des revendication précédentes, comprenant en outre les étapes de :
- mesurer (S60) l'activité antioxydante globale du produit-cible au moyen d'un dispositif de mesure (Mes) ; et
- générer (S80) :
- le signal (Sig_{typ}) représentatif d'un type du produit-cible à caractériser sur la base d'une information stockée dans une mémoire informatique (Mem) ; et
- le signal (Sigₘₑₛ) représentatif d'une activité antioxydante globale du produit-cible sur la base d'un résultat de la mesure (S60).

5. Système (Sys) de caractérisation d'un produit-cible à caractériser présentant une activité antioxydante, le système comprenant une base de données (DB) et une unité de calcul (Calc) configurés pour mettre en œuvre le procédé selon l'une quelconque des revendications 1 à 4.

## Patentansprüche

1. Verfahren (100) für eine Charakterisierung eines zu charakterisierenden Zielprodukts, das eine antioxidative Aktivität aufweist, umfassend einen Schritt (S60) zum Empfangen eines Signals (Sigₘₑₛ), das eine Messung einer gesamten antioxidativen Aktivität des zu charakterisierenden Zielprodukts darstellt, wobei das Verfahren **dadurch gekennzeichnet ist, dass** es ferner die folgenden Schritte umfasst:
- Empfangen (S80), mittels einer Datenverarbeitungseinheit (Calc), eines Signals (Sig_{typ}), das einen Typ des zu charakterisierenden Zielprodukts darstellt;
- Identifizieren (S90A) in einer Datenbank (DB) eines Referenzprodukts (Prodᵢ), das dem zu charakterisierenden Zielprodukt entspricht, auf der Basis des Signals (Sig_{typ}), das einen Typ des zu charakterisierenden Produkts darstellt,
- Abrufen (S90B) in der Datenbank (DB) von Identifikatoren (Constᵢⱼ) von antioxidativen Bestandteilen des zu charakterisierenden Zielprodukts auf der Basis des Signals (Sig_{typ}), das einen Typ des zu charakterisierenden Produkts darstellt;
- Abrufen (S90B) in der Datenbank (DB), mittels der Datenverarbeitungseinheit (Calc), (a) von Koeffizienten (Coefᵢⱼ), die jeweils die antioxidativen Aktivitäten der antioxidativen Bestandteile in dem zu charakterisierenden Zielprodukt darstellen, und (b) von individuellen antioxidativen Aktivitäten (AAᵢⱼ) der individuell berücksichtigten Bestandteile; und
- Berechnen (S90C), mittels der Datenverarbeitungseinheit (Calc), von Konzentrationen (*C'ⱼ*) der antioxidativen Bestandteile (Constᵢⱼ) in dem zu charakterisierenden Zielprodukt anhand des Signals (Sigₘₑₛ), das eine gesamte antioxidative Aktivität des Zielprodukts darstellt, Koeffizienten (Coef ᵢⱼ) und antioxidativen Aktivitäten (AAᵢⱼ) anhand der Gleichung $C {′}_{j} = \frac{{Coef}_{j} {xAA}_{GlobMes}}{{AA}_{j}}$*,* wobei *C'ⱼ* die Konzentrationen der antioxidativen Bestandteile in dem Zielprodukt darstellt, *Coefⱼ* die relativen Beiträge der Bestandteile der gesamten antioxidativen Aktivität des Referenzprodukts (Prodᵢ) angibt, *AA_{GlobMes}* die gesamte antioxidative Aktivität des Zielprodukts darstellt und *AAⱼ* die individuellen antioxidativen Aktivitäten der individuell berücksichtigten Bestandteile darstellt, und die relativen Beiträge der Bestandteile der antioxidativen Aktivität, die durch *Coefⱼ* angegeben werden, aus der Gleichung ${Coef}_{j} = \frac{{C}_{j}}{{\sum }_{j} {C}_{j}}$ abgeleitet werden, wobei *Cⱼ* die jeweiligen Konzentrationen der Bestandteile in dem Referenzprodukt (Prodᵢ) darstellt.

2. Verfahren nach Anspruch 1, wobei das Berechnen (S90) derart durchgeführt wird, dass nur antioxidative Bestandteile berücksichtigt werden, deren individuelle antioxidative Aktivitäten (AAᵢⱼ) multipliziert mit ihren jeweiligen Konzentrationen (Cᵢⱼ) 5 % der gesamten antioxidativen Aktivität des Referenzprodukts oder des zu charakterisierenden Zielprodukts überschreiten.

3. Verfahren nach Anspruch 1, wobei das Berechnen (S90) derart durchgeführt wird, dass nur antioxidative Bestandteile berücksichtigt werden, deren Summe der durch ihre jeweiligen Konzentrationen gewichteten antioxidativen Aktivitäten 80 % der gesamten antioxidativen Aktivität des Referenzprodukts oder des zu charakterisierenden Zielprodukts überschreitet.

4. Verfahren nach einem der vorstehenden Ansprüche, ferner umfassend die Schritte:
- Messen (S60) der gesamten antioxidativen Aktivität des Zielprodukts mittels einer Messvorrichtung (Mes); und
- Erzeugen (S80):
- des Signals (Sig_{typ}), das einen Typ des auf der Basis einer in einem Computerspeicher (Mem) gespeicherten Information zu charakterisierenden Zielprodukts darstellt; und
- des Signals (Sigₘₑₛ), das auf der Basis eines Ergebnisses des Messens (S60) eine gesamte antioxidative Aktivität des Zielprodukts darstellt.

5. System (Sys) für die Charakterisierung eines zu charakterisierenden Zielprodukts, das eine antioxidative Aktivität aufweist, das System umfassend eine Datenbank (DB) und eine Recheneinheit (Calc), die zum Implementieren des Verfahrens nach einem der Ansprüche 1 bis 4 konfiguriert sind.

## Claims

1. Method (100) for characterizing a target product to be characterized that exhibits antioxidant activity, comprising a step (S60) of receiving a signal (Sigₘₑₛ) which is representative of a measurement of the overall antioxidant activity of the target product to be characterized, the method being **characterized in that** it further comprises the steps of:
- receiving (S80), by means of a computer processing unit (Calc), a signal (Sig_{typ}) which is representative of a type of the target product to be characterized;
- identifying (S90A), in a database (DB), a reference product (Prodᵢ) corresponding to the target product to be characterized on the basis of the signal (Sig_{typ}) which is representative of a type of the product to be characterized,
- retrieving (S90B), in the database (DB), identifiers (Constᵢⱼ) of antioxidant constituents of the target product to be characterized on the basis of the signal (Sig_{typ}) which is representative of a type of the product to be characterized;
- retrieving (S90B), in the database (DB) by means of the computer processing unit (Calc), (a) coefficients (Coefᵢⱼ) which are representative, respectively, of the antioxidant activities of the antioxidant constituents in the target product to be characterized, and (b) individual antioxidant activities (AAᵢⱼ) of the constituents considered individually; and
- calculating (S90C), by means of the computer processing unit (Calc), the concentrations (*C'ⱼ*) of the antioxidant constituents (Constᵢⱼ) in the target product to be characterized from the signal (Sigₘₑₛ) which is representative of the overall antioxidant activity of the target product, from the coefficients (Coefᵢⱼ) and from the antioxidant activities (AAᵢⱼ) using the equation $C {′}_{j} = \frac{{Coef}_{j} \times {AA}_{GlobMes}}{{AA}_{j}}$, where *C'ⱼ* represents the concentrations of the antioxidant constituents in the target product, *Coefⱼ* indicates the relative contributions of the constituents to the overall antioxidant activity of the reference product (Prodᵢ), *AA_{GlobMes}* represents the overall antioxidant activity of the target product, and *AAⱼ* represents the individual antioxidant activities of the constituents considered individually, and the relative contributions of the constituents to the antioxidant activity, indicated by *Coefⱼ* are derived from the equation ${Coef}_{j} = \frac{{C}_{j}}{{\sum }_{j} {C}_{j}}$, where *Cⱼ* represents the respective concentrations of the constituents in the reference product (Prodᵢ).

2. Method according to claim 1, in which the calculation (S90) is performed considering only the antioxidant constituents of which the individual antioxidant activities (AAᵢⱼ), multiplied by their respective concentrations (Cᵢⱼ), exceed 5% of the overall antioxidant activity of the reference product or the target product to be characterized.

3. Method according to claim 1, in which the calculation (S90) is performed considering only the antioxidant constituents of which the sum of antioxidant activities, weighted by their respective concentrations, exceeds 80% of the overall antioxidant activity of the reference product or the target product to be characterized.

4. Method according to any of the preceding claims, further comprising the steps of:
- measuring (S60) the overall antioxidant activity of the target product by means of a measuring device (Mes); and
- generating (S80):
- the signal (Sig_{typ}) which is representative of a type of the target product to be characterized on the basis of information stored in a computer memory (Mem); and
- the signal (Sigₘₑₛ) which is representative of the overall antioxidant activity of the target product on the basis of a result of the measurement (S60).

5. System (Sys) for characterizing a target product to be characterized that exhibits antioxidant activity, the system comprising a database (DB) and a computing unit (Calc), which are configured to implement the method according to any of claims 1 to 4.
